# EUROPEAN PATENT APPLICATION

(11) **EP 4 094 664 A1**
(43) Date of publication of application: **30.11.2022**
(21) Application number: 21176573.0
(22) Date of filing: 28.05.2021
(51) Int. Cl.: A61B 1/00, A61B 1/05, A61B 1/06, A61B 1/07

(54) **AN ENDOSCOPE**

(71) Applicant: Ambu A/S, 2750 Ballerup (DK)
(72) Inventor: SØRENSEN, Morten, 2750 Ballerup (DK)
(74) Representative: AWA Denmark A/S

(57) **Abstract**

An endoscope comprising a proximal handle and a distal tip with at least one transparent front window. The distal tip comprises an electronic image capture device having an essentially rectangular defining a vertical and horizontal imaging direction. A lightguide (13) is associated with the at least one light source arranged in alignment with a centre axis (A-A) of said lightguide (13). The lightguide (13) comprises a proximal end (14) and a distal end (16) and a circumferential surface (18) extending between said proximal end (14) and said distal end (16). The circumferential surface (18) is configured to provide total internal reflection of light emitted from the light source, and said circumferential surface defines cross-sections of said lightguide (13), wherein said cross-sections comprise four corners (15) arranged around said centre axis (A-A) so as to define four edges (21) subdividing the circumferential surface (18) into four side surfaces (18). The edges (21) extend with a predetermined angle with respect to the centre axis (A-A) and said side surfaces (18) have a curvature where the angle of the surface (18) with respect to the centre axis increases from the edges (21) towards the middle of the side surface.

## Description

The present disclosure relates to insertion endoscopes, in particular but not limited to a tip part of such an endoscope.

Insertable vision devices such as endoscopes are well known for visually inspecting inaccessible places such as body cavities, e.g. human body cavities. Typically, the endoscope comprises an elongated insertion cord with a handle at the proximal end as seen from the operator and visual inspections means, such as a built in camera, at the distal end of the elongated insertion cord. Electrical wiring for the electronic vision receptor, e.g. the chip of a camera and other electronics such as LED lighting accommodated in the tip part at the distal end run along the inside of the elongated insertion cord from the handle to the tip part. Instead of using cameras, endoscopes may also be fibre-optic, in which case the optical fibres run along inside of the elongated insertion cord to the tip part.

In order to be able to manoeuvre the endoscope inside the body cavity, the distal end of the endoscope may comprise a bending section with increased flexibility, e.g. a number of articulated segments of which the tip part forms the distalmost segment. This is typically done by tensioning or slacking pull wires also running along the inside of the elongated insertion cord from the tip part through the remainder of articulated segments to a control mechanism of the handle. Furthermore, a working channel may run along the inside of the insertion cord from the handle to the tip part, e.g. allowing liquid to be removed from the body cavity or allowing the insertion of surgical instruments or the like into the body cavity.

As the name indicates, endoscopes, are used for seeing inside things, such as lungs or other human body cavities of a patient. Modern endoscopes are therefore typically equipped with at least one camera or similar image capturing device serving as an electronic vision receptor at the distal tip of the endoscope. Provided that sufficient light is present, this allows the operator to see where the endoscope is steered and to set the target of interest once the tip has been advanced thereto. This therefore normally requires illumination of the area in front of the distal tip of the endoscope, in particular the field of vision of the camera(s). One known way of achieving such illumination is to provide the above-mentioned LED lighting using one or more Light Emitting Diodes (LEDs) in conjunction with lightguides in the tip of the endoscope, as e.g. formed integrally with a front window covering also the vision receptor as mentioned in EP 3539450 disclosing a disposable endoscope incorporated herein by reference. The light distribution in the emitted light from a light source such as an optical fibre or an LED is, however, not optimal as regards the field of vision of a vision receptor such as an image sensor, a video camera or an optical fibre. In particular, an LED may spread the emitted light over a wide angle from what is ideally a point source. The vision receptor on the other hand is typically a rectangular array or matrix, with a certain width-height aspect ratio. It may therefore be problematic to have the corners of the field of vision of the vision receptor proper illuminated without overexposure of other parts. As an example, in many body cavities, in particular tubular ones, objects in the periphery of the field of vision will often be closer than those in the centre. Consequently, they will be stronger illuminated than those in the centre, which in turn leads to overexposure of the image at the periphery and underexposure in the centre where the object of interest is often likely to be. The opposite, however, may also be the case so that the centre is overexposed while corners of the field of vision is underexposed. This could be the case in a more open space such as in the bladder or in the stomach.

JP-8-122633 deals with the light distribution to the corners of the field of view of a reusable endoscope. However, rather than using lightguides in front of LEDs JP-8-122633 uses relatively complicated lens systems in front of optical fibres, providing the light at the tip of the endoscope.

Based on this prior art, it is the object of the present disclosure to provide a tip part of an endoscope which overcomes at least some of the above problems.

According to a first aspect of the disclosure this object is achieved by an endoscope comprising a proximal handle and a distal tip with at least one transparent front window, said distal tip being arranged at the distal end of an insertion cord extending from said proximal handle, said distal tip comprising an electronic image capture device having an essentially rectangular shape defining a vertical and horizontal imaging direction, the distal tip comprises at least one light source, a lightguide is associated with the at least one light source arranged in alignment with a centre axis of said lightguide, where the lightguide comprises a proximal end and a distal end and a circumferential surface extending between said proximal end and said distal end, wherein said circumferential surface is configured to provide internal reflection of light emitted from the light source, and said circumferential surface defines cross-sections of said lightguide, wherein said cross-sections comprise four corners arranged around said centre axis so as to define four edges subdividing the circumferential surface into four side surfaces, where said edges extend with a predetermined angle with respect to the centre axis and said side surfaces having a curvature where the angle of the surface with respect to the centre axis increases from the edges towards the middle of the side surface.

According to a second aspect of the disclosure the object is achieved by a system comprising a display device and an endoscope according to the first aspect of the disclosure connectable to the display device.

According to a first embodiment of the first aspect of the disclosure the edges are arranged with a 90° spacing around said centre axis. This provides a high degree of symmetry around the light source which is ideally but not in practice a point source aligned with the centre axis, thus providing good control of the light emission through and from the lightguide.

According to another embodiment of the first aspect of the invention the side surfaces between said edges are smooth, i.e. without any discontinuities such as concave corners between the convex corners forming the edges. This further improves the control of light emission through and from the lightguide and facilitates manufacturing of the lightguide as it is easier to e.g. injection mould.

According to a further embodiment of the first aspect of the disclosure the distal end face of the of the lightguide constitutes the front window or part of the front window. This may further improve the control of the light emitted from the endoscope directly from the lightguide rather than through a front windowpane or similar object without lightguide properties irrespective of the front windowpane is integral with the lightguide or not.

An endoscope according to any one of the preceding claims, wherein the light guide and the window is a single-piece integrally moulded component. This makes the manufacturing cost-efficient.

According to yet a further preferred embodiment of the first aspect of the disclosure, the angle of the edges with respect to the centre axis is between 0° and 3°, preferably approximately 1°. This allows for easy manufacture of the lightguide by e.g. injection moulding without inhibiting the desired optical properties.

According to yet another preferred embodiment of the first aspect of the disclosure the maximum angle of the side surfaces with respect to the centre axis is in the interval from 2° to 10°, preferably approximately 5°. This provides a proper curvature to reflect some of the light incident on the side surfaces more towards the corners as seen in the cross-sectional plane and hence emanate in a corresponding direction from the lightguide into the field of view of the image capture device.

The disclosure will now be made in greater detail based on non-limiting exemplary embodiments and with reference to the drawings, on which:
Fig. 1 shows an isometric view of the tip part of an endoscope implementing the disclosure,
Fig. 2 shows the inside of the tip part of Fig. 1 seen from the proximal end,
Figs. 3a-3c shows a first embodiment of a lightguide that may be implemented in the tip part of Fig. 1,
Figs. 3d-3f show emission diagrams for the first embodiment,
Figs. 4a-4c shows a second embodiment of a lightguide that may be implemented in the tip part of Fig. 1,
Figs. 4d-4f show emission diagrams for the second embodiment,
Figs. 5a-5c shows a third embodiment of a lightguide that may be implemented in the tip part of Fig. 1,
Figs. 5d-5f show emission diagrams for the third embodiment,
Figs. 6a-6c shows a fourth embodiment of a lightguide that may be implemented in the tip part of Fig. 1,
Figs. 6d-6f show emission diagrams for the fourth embodiment,
Figs. 7a-7c shows a fifth embodiment of a lightguide that may be implemented in the tip part of Fig. 1,
Figs. 7d-7f show emission diagrams for the fifth embodiment,
Fig. 8 shows a system of a display unit and an endoscope with a tip part according to the disclosure connectable to the display unit.

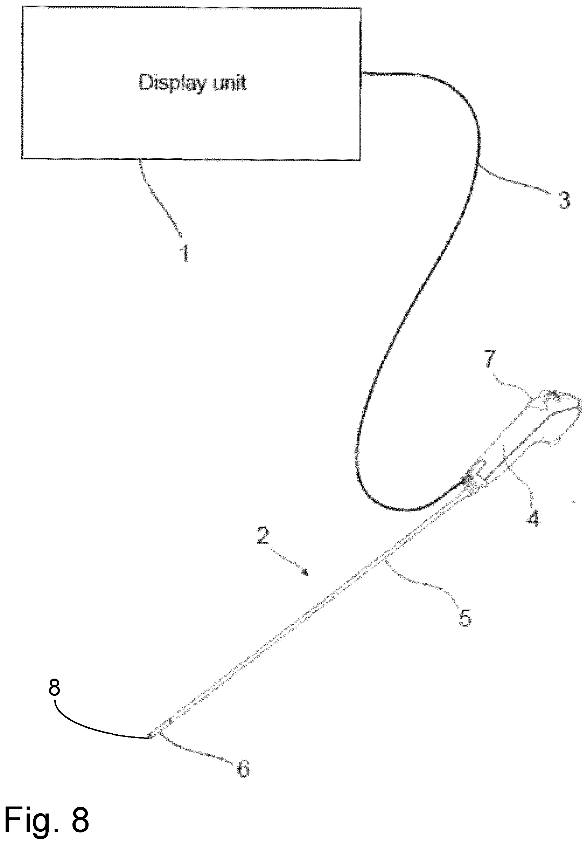

Turning first to Fig. 8, a system according to the second aspect of the disclosure is shown. The system comprises a display unit 1, such as a monitor with a screen, and an endoscope 2 connectable thereto via a wireless connection or a cable 3, as shown. The endoscope 2 is preferably a forward looking, disposable, i.e. single use, endoscope that is to be discarded after use in a patient, rather than cleaned, sterilized and reused. The endoscope 2 comprises a handle 4 at the proximal end adapted to be gripped by the hand of an operator, and a bendable insertion cord 5 extending towards the distal end of the endoscope 2 and adapted to be inserted into a patient. At the distal end of the insertion tube the endoscope comprises a tip part 8 according to the disclosure connected to the remainder of the insertion cord 5, i.e. a main tube part, via an articulated bending section 6. The articulated bending section 6 is highly bendable. The bending motion of the bending section is controlled by the user using an operating member 7 such as knob or a lever via control cables (not visible) connected to the tip part 8 or the distal end of the bending section 6. The bending section 6 comprises a thin covering sheath, and the inner details are thus not visible in Fig. 8.

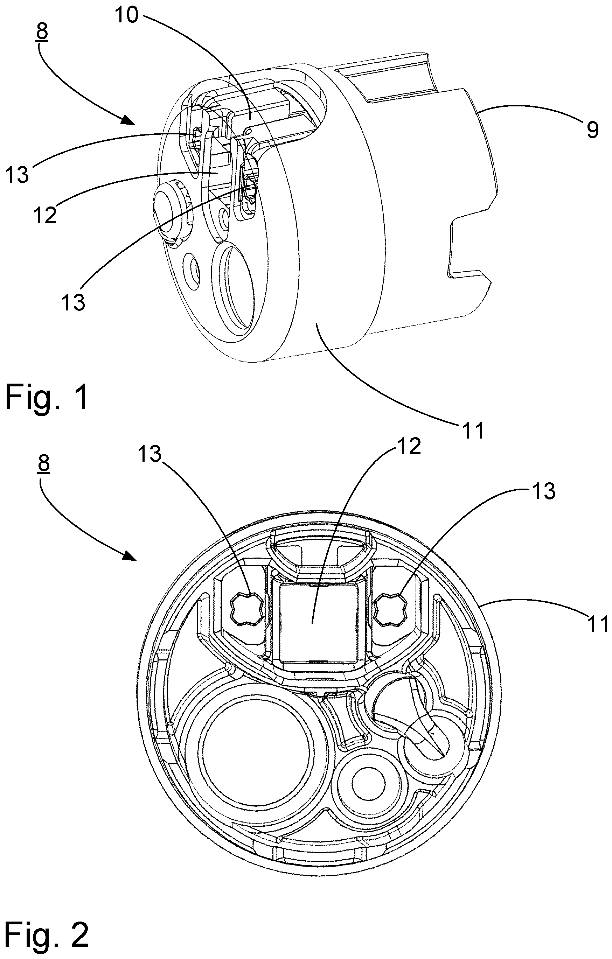

Turning now to Fig. 1, the housing 9 a tip part 8 of the endoscope 2 is shown in greater detail. The housing 9 may be moulded integrally in a two-stage two component injection moulding process so as to have a transparent housing part 10 and an opaque housing part 11. The transparent housing part 10 comprises a front window part 12 preferably covering, as seen from the distal end, an electronic imaging device (not shown) provided in the tip housing 9. The front window part 12 furthermore comprises integrally formed lightguides 13 adapted to guide and distribute light from light sources, such as LEDs (not shown), also provided in the tip housing 9, in order to provide illumination for the imaging device. It is, however, not excluded that the imaging device is provided with its own front window or lens, and that the lightguides 13 themselves constitute a front window part 12.

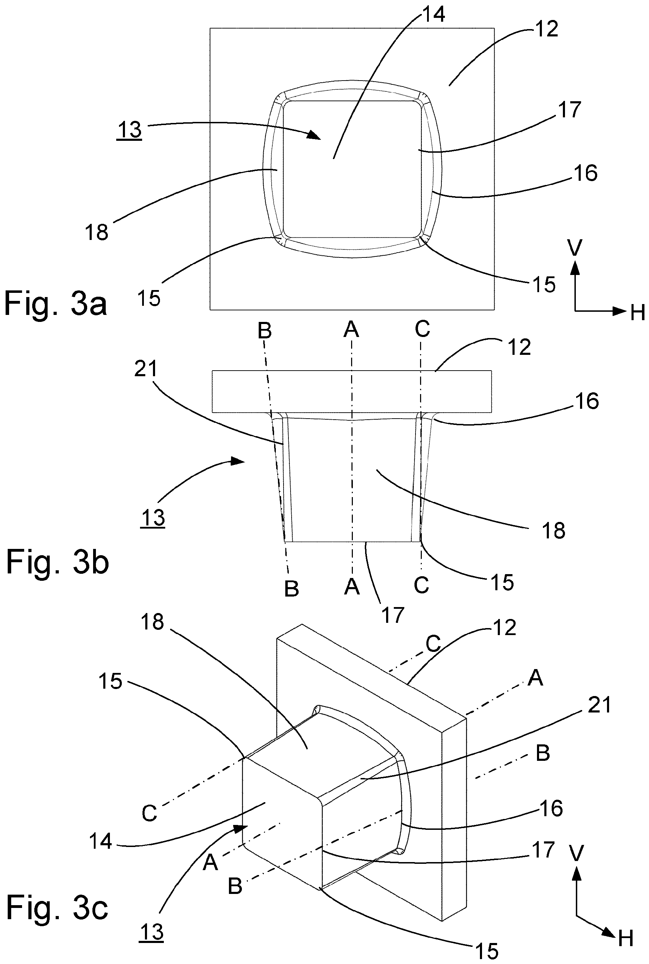

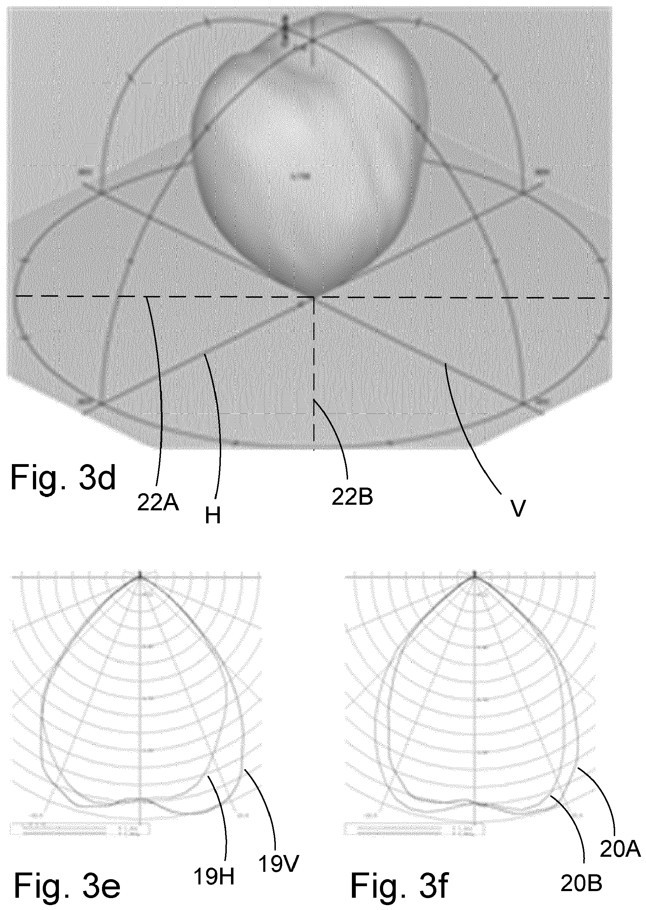

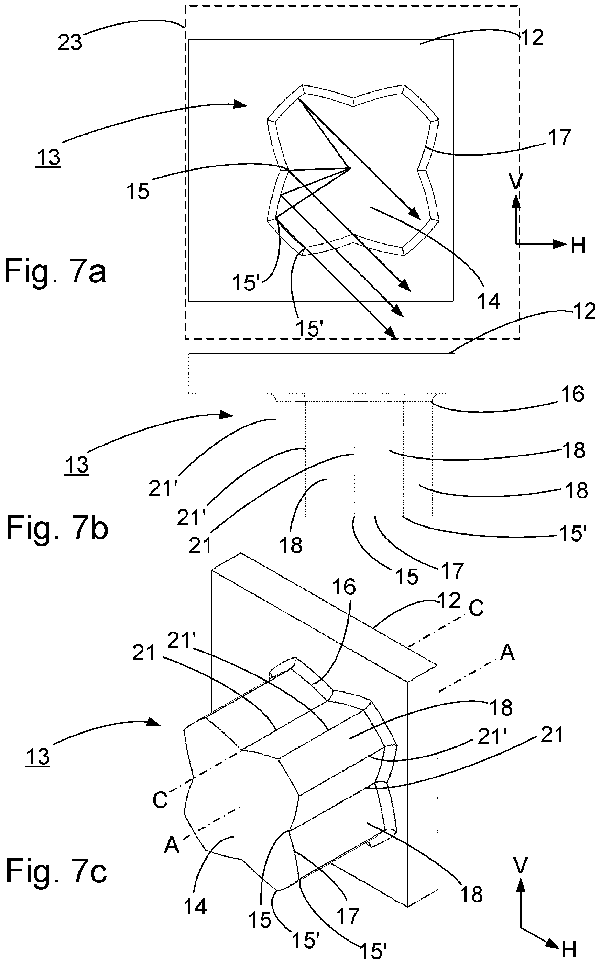

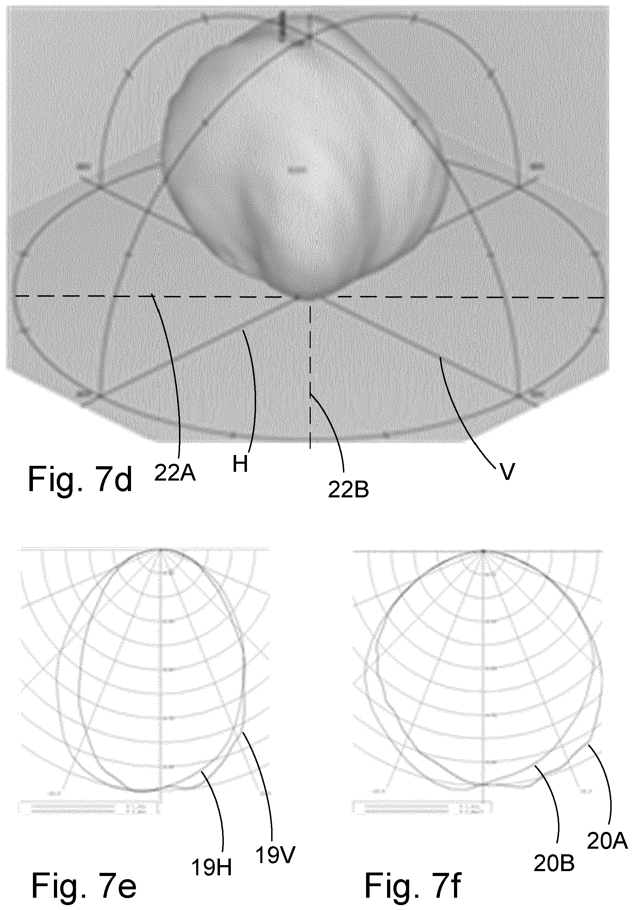

As can be seen the lightguides 13 shown in Figs. 1 and 2 correspond specifically to the fifth embodiment shown in Figs. 7a-7c, but may in other embodiments of the housing 9 be substituted with other lightguides 13, such as corresponding to those depicted in Figs. 3 to 6, as they all improve the light distribution according to the disclosure.

Turning now to Figs. 3a to 3c, a first embodiment of a lightguide 13 is shown as seen from the proximal end corresponding to the direction of Fig. 2. For simplicity, the front window part 12 is just schematically shown as a square but could of course have the full complexity of the front window part 12 of Figs. 1 and 2. The front window part 12 may be integrally moulded with the lightguide 13 but could also be a separate item. Furthermore, the end face of the lightguide 13 could itself constitute the front window. The same applies to other embodiments described below.

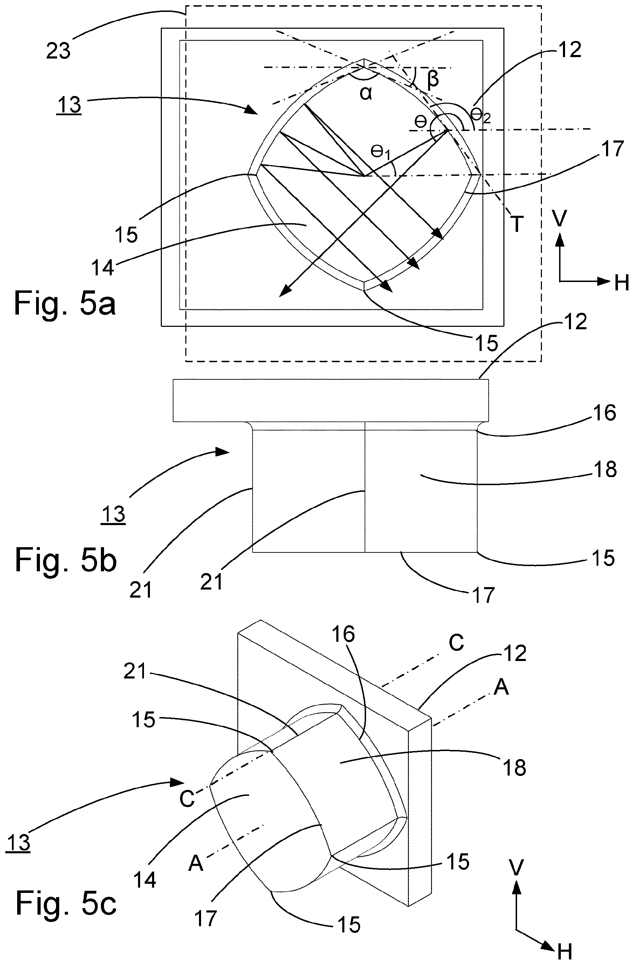

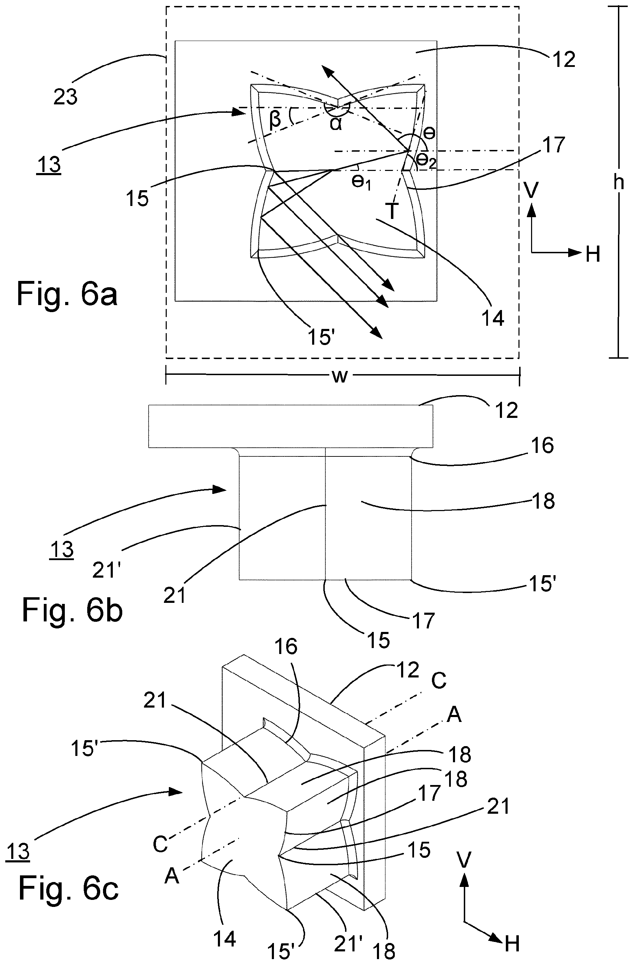

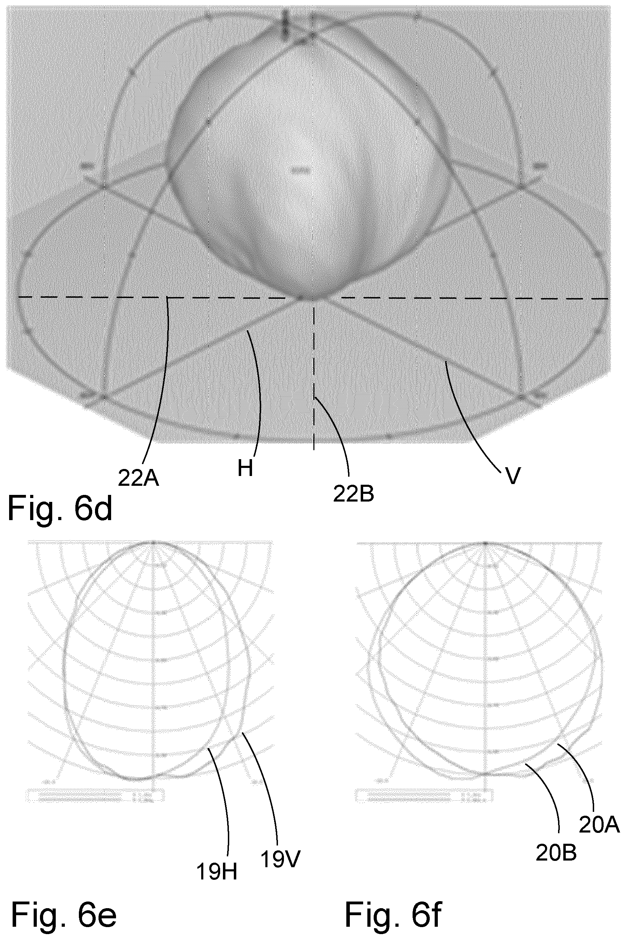

As can be seen, the body of the lightguide 13 is roughly the shape of a frustum of a four-sided pyramid. The base is contiguous with the front window part 12 and the top, i.e. the proximal end, comprises a plane surface 14 adapted to receive and be in good contact with the emission side of an LED component. The plane surface 14 at the top is generally rectangular with four straight sides 17 except at the corners 15, which are slightly rounded for manufacturing reasons. Being a square, the corners are spaced with 90° spacing about a centre axis A-A. The sides are aligned with the horizontal imaging direction H and vertical imaging direction V defined by the image sensor or receptor of the electronic image capture device 23, schematically indicated with broken lines in Figs. 5a, 6a and 7a and the corresponding rectangular display on the display unit. In the illustrated examples the image sensor 23 is assumed to be square meaning that the width w and the height h, as indicated on Fig. 6a, are equal, i.e. that the aspect ratio h/w is 1. However, as will be explained below, other aspect ratios are also possible, within the scope of invention. The cross-section at the base is not entirely rectangular but rather somewhat cushion-shaped with four convexly curved sides 16. As far as the lightguide 13 itself is considered, it ends at the base with the sides 16 because the front window part 12 beyond that distal end of the lightguide does not contribute with any internal reflection. In practice there will be some curvature in the transition between base formed by the sides 16 of the lightguide 13 and the front window part 12, which is also not considered to be a part of the lightguide. The same applies to all other embodiments shown in the figures.

The four side surfaces 18 which together with the rounded corners form the circumferential surface of the lightguide 13 are each smooth surfaces without discontinuities with a curvature that (as seen from the outside) increases in convexity from the top towards the base. The four edges 21 formed by the corners are preferably perpendicular to the exit surface of the front window part 12, as indicated by the axis C-C, or as close to perpendicular as the manufacturing methods allow. That is ideally to say approximately 0° angle to the centre axis A-A about which the lightguide 13 exhibits a fourth order of symmetry. Some deviation may be acceptable for manufacturing reasons and the angle may thus be anywhere between 0° and 3°, preferably approximately 1°.

From the corner, when moving towards the middle of the side surface, the angle of the surfaces increase slightly from approximately 0° to a maximum angle of the side surfaces with respect to the centre axis A-A as indicated by the axis B-B. Because the maximum angle increases away from each corner the maximum angle is found midway on the surface 18 between two edges 21 defined by the corners. This maximum angle is preferably in the interval from 2° to 10°, but is always larger than the angle of the corners with respect to the centre line A-A.

With properly selected transparent materials this shape of the lightguide provides total internal reflection of the light from the light source, but because of the curvature of the side surfaces more light is emitted in the directions away from the corners of the lightguide 13. That is to say, the light is collimated more at the sides where the angles are higher than in the corners where the angles are lower, as described in EP 3539450. Suitable transparent materials could include Polycarbonate, Cyclic Olefin Polymer, Cyclic Olefin Copolymer, Styrene-Butadiene Copolymer, Silicone or Polystyrene. If the orientation of the edges 17 of the square shape of the plane surface 14 is aligned with the sides of the normally rectangular image sensors of the imaging device, more light will be available in the corners of the field of view. This can be seen from the example shown in Figs. 3d to Fig. 3f, where the curves 20A, 20B indicate that more light is emitted in the direction corresponding to the dashed axes 22A, 22B than in the direction corresponding to the fully drawn axes H, V corresponding to the curves 19H, 19V. Deviations may occur as the emission from the LEDs is not necessarily coincident with the centre of the emission surface of the LED component, in turn involving a potential misalignment with the centre of the lightguide 13 that needs to be minimized during manufacture.

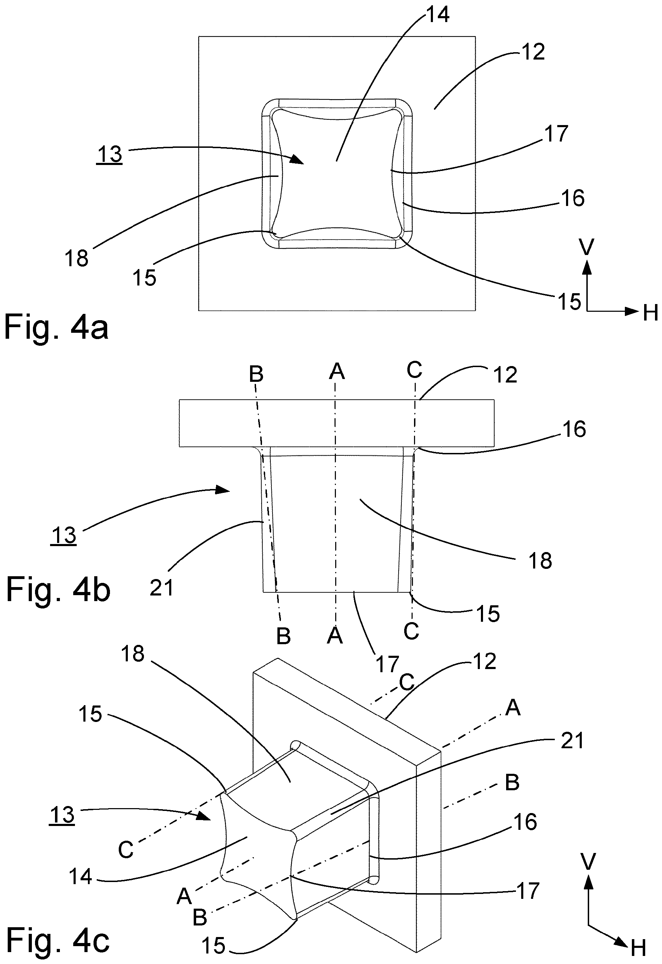

Turning now to Fig. 4a to 4c, a second embodiment of a lightguide 13 is shown as seen from the proximal end corresponding to the direction of Fig. 2. Again, for simplicity, the front window part 12 is just schematically shown as a square but could of course have the full complexity of the front window part 12 of Figs. 1 and 2.

As can be seen, the body of the lightguide 13 is roughly the shape of a frustum of a four-sided pyramid. The base is preferably contiguous with the front window part 12 and the top, i.e. the proximal end, comprises a plane surface 14 adapted to receive and be in good contact with the emission side of a LED component. In this and other embodiments, the base could, however, also constitute the front window, or the front window could be a separate part. The plane surface 14 at the top is generally rectangular, however with four concavely curved edges 17, meeting at the corners 15, which are also in this embodiment slightly rounded for manufacturing reasons. The corners are spaced with 90° spacing about a centre axis A-A, so as to provide a fourth order of rotational symmetry. In this second embodiment the cross-section at the base is square with straight sides 16 and rounded corners. The sides are aligned with the horizontal imaging direction H and vertical imaging direction V defined by the image sensor and the corresponding rectangular display on the display unit.

The four side surfaces 18 which together with the rounded corners form the circumferential surface of the lightguide 13 are each smooth surfaces without discontinuities with a curvature that, like the first embodiment, increases in convexity from the top towards the base or, depending on how you express it, decreases in concavity. Like the first embodiment, the four edges 21 formed by the corners are preferably perpendicular to the exit surface of the front window part 12, as indicated by the axis C-C, or as close to perpendicular as the manufacturing methods allow. That is ideally to say approximately 0° angle to the centre axis A-A about which the lightguide 13 exhibits a fourth order of symmetry. Some deviation may be acceptable for manufacturing reasons and the angle may thus be anywhere between 0° and 3°, preferably approximately 1°.

From the corner, the angle of the surfaces increases slightly from approximately 0° to a maximum angle of the side surfaces with respect to the centre axis A-A. Because the maximum angle increases away from each corner the maximum angle is found midway on the surface 18 between two edges 21 defined by the corners as indicated by the axis B-B. This maximum angle is preferably in the interval from 2° to 10°, but is always larger than the angle of the corners with respect to the centre line A-A.

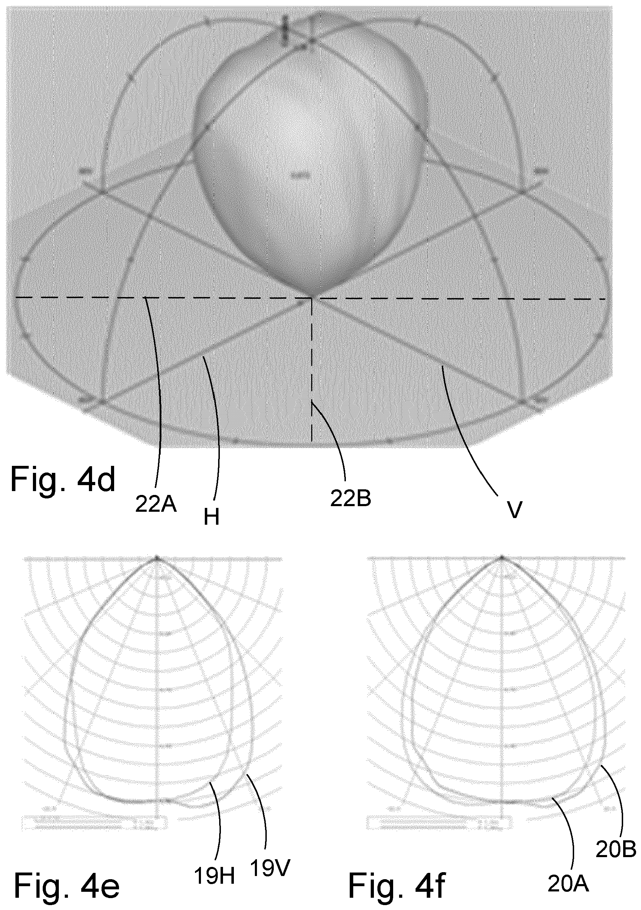

With properly selected transparent materials this shape of the lightguide provides total internal reflection of the light from the light source, but because of the curvature of the side surfaces, the light is collimated more at the sides where the angles are higher than in the corners where the angles are lower, and accordingly light is emitted more to the sides than forwardly at the corners of the lightguide 13 than at the side surfaces where the angles are larger. If the orientation of the edges 17 of the plane surface 14 is aligned with the sides of the normally rectangular image sensors of the imaging device, more light will be available in the corners of the field of view. This can be seen from the example shown in Figs. 4d to Fig. 4f, where the curves 20A, 20B indicate that more light is emitted in the direction corresponding to the dashed axes 22A, 22B than in the direction corresponding to the fully drawn axes H, V corresponding to the curves 19H, 19V. Deviations may occur as the emission from the LEDs is not necessarily coincident with the centre of the emission surface of the LED component, in turn involving a potential misalignment with the centre of the lightguide 13 that needs to be minimized during manufacture.

Turning now to Figs. 5a to 5c an alternative embodiment of the lightguide 13 adapted for increasing light emission towards the corners of the field of view of the imaging device is shown. The lightguide 13 in this embodiment is not a truncated pyramid but rather a column. That is to say the base and the top 14 have essentially the same shape and area. Accordingly, the sides 18 and the edges 21 are perpendicular to the end surface, except for whatever deviation may be necessary for other reasons, i.e. small deviation in the interval from 0° to 3°. Such reasons may include manufacturing reasons but also collimation, e.g. to generally keep as much light as possible withing the field of view of the imaging device.

As can be seen the areas of top 14 and base has four corners 15 but is not quite square, but rather somewhat cushion-shaped, i.e. with a convex curvature as seen from the outside. This again applies to the entire surface outer 18 between the corners.

However, as seen from the inside of the lightguide 13 where incident light from the LED is subject to internal reflection the light is thus reflected of a concave surface. That is to say, the surface as seen from the inner of the light guide 13 is a concave mirror. Since, as will be understood, reflection is the issue, all references to convex and concave corners and surfaces will be made accordingly, i.e. as seen from the inside of the light guide 13, rather than in the conventional way for polygons in geometry. In this respect, corners will be understood as convex if the angle at which the surface meet, is larger than 180°, as seen from the inside, and concave if the angle with which they meet is smaller than 180°, as seen from the inside. In other words a convex corner points towards the centre of the light guide 13, and concave corners point away from the centre of the light guide 13. With the corners 15 arranged as two pairs of opposing concave corners 15 one pair opposing each other in the vertical direction V and the other pair opposing each other in the horizontal direction H, as defined with respect to the image receptor and display, it is possible to reflect more light towards the corners of the image than the sides of the field of view of the square image receptor as illustrated in Fig 5a. As can be seen the curvature of the surface 18 is concave and so selected that light impinging midway on the surface is reflected straight back towards the centre line A-A whereas light impinging closer to and at the corners 15 are reflected back at an angle so that all light is generally directed more in a direction between the horizontal and vertical directions. The curvature may be selected differently from that shown, but ideally at the corners the angle β of the tangent to the surface with respect to vertical or horizontal direction should be 22.5° or at least in the interval between 20° and 25° in order to achieve the desired reflection at an angle of 45° with respect to vertical or horizontal as illustrated. In the example illustrated in Figs. 5a to 5c, this results in a concave corner 15 as seen from within the lightguide 13 with an angle α of 135° or at least between 125° and 145°.

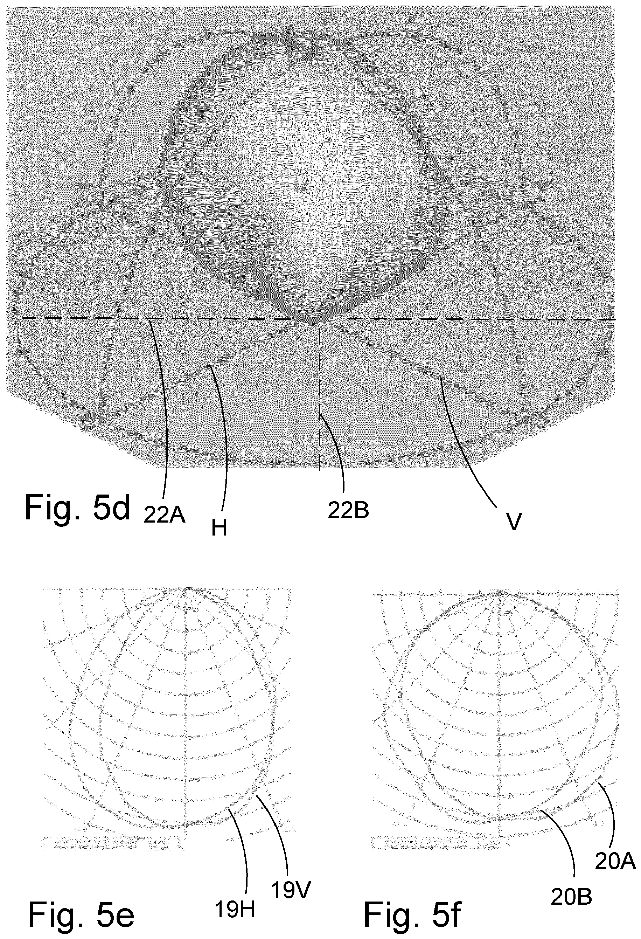

Thus, with opposing corners 15 and resulting edges 12 arranged along the vertical and horizontal direction and suitably selected angles of the corners 15, more light will be available in the corners of the field of view of the imaging device. This can be seen from the example shown in Figs. 5d to Fig. 5f, where the curves 20A, 20B indicate that more light is emitted in the direction corresponding to the dashed axes 22A, 22B than in the direction corresponding to the fully drawn axes H, V corresponding to the curves 19H, 19V. Deviations may occur as the emission from the LEDs is not necessarily coincident with the centre of the emission surface of the LED component, in turn involving a potential misalignment with the centre of the lightguide 13 that needs to be minimized during manufacture.

Irrespective of whether the corners are arranged exactly opposite each other and whether the image sensor is generally rectangular i.e. height h differs from width w, the curvature between two neighbouring corners is so selected that the reflection angle θ with respect to horizontal H is so selected that the reflection angle (θ) is either ± arctan (h/w), or ±(180° - arctan (h/w)). That is to say if the aspect ratio of the image sensor is not 1, the angles towards the corner are not 45°, the curvature is still adapted to reflect light towards the corners, and does thus not deviate from the scope of the disclosure.

As illustrated in Fig. 5a this can be achieved if the tangent T to the reflecting surface is at an angle θ₂ to horizontal, where θ₂ is so selected that θ = 2θ₂ - θ₁, where θ₁ is the emission angle of a light ray emanating from the centre axis with respect to said horizontal direction H.

In Figs. 6a to 6c another embodiment of the lightguide 13 adapted for increasing light emission towards the corners of the field of view of the imaging device is shown. Like the lightguide illustrated in Figs. 5a-5c, the lightguide 13 in this embodiment is also not a truncated pyramid but rather a column. That is again to say the base and the top 14 have essentially the same shape and area. Accordingly, the sides 18 and the edges 21 are perpendicular to the end surface, except for whatever deviation may be necessary for other reasons, i.e. small deviation in the interval from 0° to 3°. Such reasons may include manufacturing reasons but also collimation, e.g. to generally keep as much light as possible withing the field of view of the imaging device. As can be seen the areas of top 14 is also not quite square, and has not only four corners 15 arranged as two pairs of opposing corners 15 one pair opposing in the vertical direction V and the horizontal direction H, as defined with respect to the image receptor and display, but four additional corners 15'. These additional corners are essential concave right-angled corners as seen from the inside of the lightguide 13.

As will be noticed, the opposing corners 15 in the vertical direction and the horizontal direction are not concave corners 15 but instead convex corners 15.

As seen from the inside of the lightguide 13 incident light from the LED is subject to internal reflection the light is also in this embodiment reflected off a concave surface 18, of which there are eight. The curvature of the surface 18 is so selected that no light impinging on any of the surfaces 18 is reflected straight back towards the centre line A-A but all reflected back at an angle so that all light is generally directed more in a direction between the horizontal and vertical directions.

The curvature may be selected differently from that shown, but ideally at the corners the angle β of the tangent to the surface with respect to vertical or horizontal direction should be 22.5° (or -22.5°) in order to achieve the desired reflection at an angle of 45 ° with respect to vertical or horizontal as illustrated for the square image receptor 23. In the example illustrated in Figs. 6a to 6c, this results in a convex corner 15 as seen from within the lightguide 13 with an angle α of 225°.

Thus, with opposing corners 15 and resulting edges 12 arranged along the vertical and horizontal direction and suitably selected angles of the corners 15, more light will be available in the corners of the field of view of the imaging device. This can be seen from the example shown in Figs. 6d to Fig. 6f, where the curves 20A, 20B indicate that more light is emitted in the direction corresponding to the dashed axes 22A, 22B than in the direction corresponding to the fully drawn axes H, V corresponding to the curves 19H, 19V. Deviations may occur as the emission from the LEDs is not necessarily coincident with the centre of the emission surface of the LED component, in turn involving a potential misalignment with the centre of the lightguide 13 that needs to be minimized during manufacture. It will be noticed that the light emission towards the corners of the field of view is better for this embodiment than for the previously described embodiment, but that this comes at the expense of higher complexity, such as *inter alia* the sharp corners 15', leading to more complicated tooling in manufacture.

Similar to the previously described embodiment, irrespective of whether the corners are arranged exactly opposite each other, the curvature between two neighbouring corners is so selected that the reflection angle θ with respect to horizontal H is so selected that the reflection angle (θ) is either ± arctan (h/w), or ±(180° - arctan (h/w)). That is to say, if the aspect ratio of the image sensor 23 differs from 1 and the angles towards the corner are not 45°, the curvature is still adapted to reflect light towards the corners. As can be seen from Fig. 6a, this again can be achieved when the tangent T to the reflecting surface is at an angle θ₂ to horizontal, where θ₂ is so selected that θ = 2θ₂ - θ₁, where θ₁ is the emission angle of a light ray emanating from the centre axis with respect to said horizontal direction H.

A further embodiment of the lightguide 13, which can be seen as a hybrid between the two previously described embodiments is shown in Figs. 7a to 7c avoids these sharp corners. This embodiment, which as the two previously described embodiments, comprises only concave inner surfaces 18 for total internal reflection, but has another four additional corners 15' compared to figs. 6a to 6c and accordingly twelve corners 15, 15' in total and a corresponding number of concave surfaces 18 for total internal reflection. The shown embodiment is, like the previous ones especially adapted for a square image receptor 23, but is like those previous embodiments, also generally applicable to other aspect ratios without deviating from the disclosure.

It thus has not only four convex corners 15, arranged as two pairs of opposing corners 15, one pair opposing in the vertical direction V and the horizontal direction H, as defined with respect to the image receptor and display, but eight additional corners 15'. These additional corners 15' are essential concave corners as seen from the inside of the lightguide 13, but with a much more obtuse angle than in the previously described embodiment. The additional surface 18 provided this way provides internal reflections similar to the ones achieved in the embodiment of Figs. 5a to Fig. 5c, i.e. more backwards in direction of the centre line A-A from which it was ideally emitted.

Accordingly, the curvature between neighbouring corners is so selected that the reflection angle θ with respect to horizontal H is so selected that the selected that the reflection angle (θ) is either ± arctan (h/w), or ±(180° - arctan (h/w)). So also here, if the aspect ratio of the image sensor 23 differs from 1 and the angles towards the corner are not 45°, the curvature is still adapted to reflect light towards the corners.. Although not shown, this is also in this case fulfilled when the tangent T to the reflecting surface is at an angle θ₂ to horizontal, where θ₂ is so selected that θ = 2θ₂ - θ₁, where θ₁ is the emission angle of a light ray emanating from the centre axis with respect to said horizontal direction H.

Further corners 15', i.e. higher multiples of four than eight can be envisaged.

The curvature may be selected differently from that shown, but ideally at the convex corners 15 as seen from within the lightguide 13 are also with an angle of 225° in this embodiment.

In general, for the above embodiments, especially adapted for image sensors with square imaging surface, it should be noted that arctan (1/1) is 45. Accordingly, the reflection angle may also be expressed as: θ = -45° + N · 90°, where N is an integer.

As mentioned above, if the aspect ratio differs from 1, as in many commonly used rectangular image sensor formats, the angles towards the corners will differ.

Thus if h/w = 9/16, the angles θ will be ± arctan (9/16), or ±(180° - arctan (9/16)) or approximately: 29.4°=(arctan(9/16)), 150.6°=(180°-arctan(9/16)), 209.4° (-150.6°) or 330.6° (-29.4°).

Similarly, if h/w = 3/4, the angles θ will be ± arctan (3/4), or ±(180° - arctan (3/4)) or approximately: 36.9°=(arctan(3/4)), 143.1°=(180°-arctan(3/4)), 216.9° (-143.1°) or 323.1° (-36.9).

It may furthermore be envisaged that features of the embodiments of Fig. 3 and Fig. 4 may be combined with the features of the embodiments of Figs. 5 to Fig. 7 and vice versa.

## Claims

1. An endoscope comprising a proximal handle and a distal tip with at least one transparent window, said distal tip being arranged at the distal end of an insertion cord extending from said proximal handle, said distal tip comprising
an electronic image capture device having an essentially rectangular receptor defining a vertical and horizontal imaging direction,
the distal tip comprises at least one light source,
a lightguide is associated with the at least one light source, where
the lightguide comprises a proximal end and a distal end and a circumferential surface surrounding a centre axis and extending between said proximal end and said distal end, wherein
said circumferential surface is configured to provide internal reflection of light emitted from the light source, and said circumferential surface defines cross-sections of said lightguide, wherein said cross-sections comprise four corners arranged around said centre axis so as to define four edges subdividing the circumferential surface into four side surfaces, where
said edges extend with a predetermined angle with respect to the centre axis and said side surfaces having a curvature where the angle of the surface with respect to the centre axis increases from the edges towards the middle of the side surface.

2. An endoscope according to claim 1, wherein the edges are arranged with a 90° spacing around said centre axis.

3. An endoscope according to claim 1, wherein said side surfaces between said edges are smooth.

4. An endoscope according to any one of the preceding claims, wherein the distal end face of the of the lightguide constitutes the front window or part of the front window.

5. An endoscope according to any one of the preceding claims, wherein the light guide and the window is a single-piece integrally moulded component.

6. An endoscope according to any one of the preceding claims, wherein the angle of the edges with respect to the centre axis is between 0° and 3°, preferably approximately 1°.

7. An endoscope according to any one of the preceding claims wherein the maximum angle of the side surfaces with respect to the centre axis is in the interval from 2° to 10°, preferably approximately 5°.

8. An endoscope according to anyone of the preceding claims wherein, the at least one light source is arranged in alignment with the centre axis of the lightguide.

9. A system comprising a display device and an endoscope according to any one of the preceding claims connectable to said display device.
